# EUROPÄISCHE PATENTSCHRIFT

(11) **EP 2 420 214 B1**
(45) Veröffentlichungstag und Bekanntmachung des Hinweises auf die Patenterteilung: **26.02.2014**
(21) Anmeldenummer: 10008690.9
(22) Anmeldetag: 19.08.2010
(51) Int. Cl.: A61F 13/02

(54) **Wundauflage, enthaltend Schaumstoff und Salbengrundlage zur Unterdrucktherapie**
Wound dressing comprising foam and salve basis for negative pressure therapy
Pansement comprenant une mousse et un excipient de pommade destiné à la thérapie par pression négative

(43) Veröffentlichungstag der Anmeldung: 22.02.2012
(60) Teilanmeldung: 13194748.3
(73) Patentinhaber: Paul Hartmann AG, 89522 Heidenheim (DE)
(72) Erfinder: Croizat, Pierre, 89542 Herbrechtingen (DE); Eckstein, Axel, 89522 Heidenheim (DE)
(74) Vertreter: Aechter, Bernd

(56) Entgegenhaltungen:
- US-A1- 2008 208 147

## Beschreibung

Die Erfindung betrifft eine Vorrichtung zur Unterdrucktherapie von Wunden umfassend, (a) ein Abdeckmaterial zum luftdichten Verschließen eines Wundraums; (b) gegebenenfalls ein Mittel zum Anschließen einer Unterdruckquelle; und (c) eine Wundauflage, enthaltend (c1) einen offenzelligen Schaumstoff, der mit (c2) einer Salbengrundlage benetzt ist, sowie ein Verfahren zur Herstellung einer entsprechenden Wundauflage. Ferner betrifft die Erfindung die Verwendung eines offenzelligen Schaumstoffs, der mit einer Salbengrundlage benetzt ist, zur Anwendung als Wundauflage in der Unterdrucktherapie von Wunden.

Unter einer Wunde wird die Trennung des Zusammenhangs von Geweben der Körperhülle bei Menschen oder Tieren verstanden. Sie kann mit einem Verlust an Substanz verbunden sein.

Vorrichtungen zur Unterdrucktherapie von Wunden sind im Stand der Technik bekannt.

So beschreibt beispielsweise die WO 1993/009727 A1 eine Vorrichtung zur Förderung der Wundheilung durch die Applikation von Unterdruck auf dem die Wunde aufweisenden und die Wunde umgebenden Hautbereich. Die Vorrichtung gemäß WO 1993/009727 A1 umfasst eine Vakuumeinrichtung zur Erzeugung des Unterdrucks, eine luftdichte Abdeckung der Wunde, welche mit der Vakuumeinrichtung in einer funktionellen Verbindung steht, sowie eine Wundauflage zur Positionierung an der Wunde innerhalb der luftdichten Abdeckung.

Geräte zur Unterdrucktherapie von Wunden sind kommerziell erhältlich, beispielsweise das V.A.C.^{®}-Gerät der Firma KCI. Bei kommerziell erhältlichen Geräten wird oftmals eine Wundauflage eingesetzt, welche einen offenzelligen Polymer-Schaumstoff, wie beispielsweise Polyvinylalkohol (PVA) oder Polyurethan (PU), enthält.

US 2008/208147 A1 (ARGENTA LUIS O [US] ET AL) offenbart eine Vorrichtung zum Anlegen von verringertem Druck für die Unterdruckbehandlung eines Gewebes. Die Vorrichtung umfasst eine Abdeckung zum Abdecken der Wunde, wobei die Abdeckung so ausgelegt ist, dass sie einen Unterdruck, welcher von einer Unterdruckquelle erzeugt wird, auf der wundzugewandten Seite aufrecht erhalten kann.

Die kommerziell üblichen Schaumauflagen werden, abhängig vom angelegten Unterdruck, unterschiedlich stark komprimiert. Dadurch kann eine Verengung der für den Abtransport des Wundexsudats nötigen Durchgänge entstehen. Weiterhin kann eine Verklebung des Schaums mit dem Wundgrund auftreten. Neu gebildetes Gewebe kann in den Schaum einwachsen. Dieses Problem ist eine bekannte Komplikation bei der Unterdrucktherapie von Wunden (FDA complaint data base). Um dieses Problem zu lösen, werden häufig zusätzliche Wundkontaktschichten zwischen Schaum und Wundgrund eingebracht, beispielsweise eine Folie (siehe z.B. WO2001/85248). Diese zusätzlichen Wundkontaktschichten vermindern aber den Durchfluss von Exsudat.

Verklebter Schaum muss zudem beim Wechseln des Verbands aufwendig entfernt werden, beispielsweise durch Spülen mit Ringerlösung. In den Schaum eingewachsenes Gewebe kann beim Verbandwechsel zu einer Gewebetraumatisierung und damit zur Störung der Wundheilung führen.

Beim Einsatz herkömmlicher Wundauflagen können zudem Schaumstoffpartikel in die Wunde gelangen. Dieses Problem wird noch verstärkt, wenn die Wundauflage vor der Anwendung auf die Größe der Wunde zugeschnitten wird, da dann insbesondere lose Schaumstoffpartikel an den Schnitträndern erzeugt werden. Auch hat sich gezeigt, dass die üblichen Polymer-Schaumstoffe teilweise mit der Abdeckfolie verkleben. Ein Verkleben der Wundauflage mit der Abdeckfolie wirkt sich insbesondere beim Entfernen des Verbandes nachteilig aus.

Der vorliegenden Erfindung liegt die Aufgabe zugrunde, die Unterdruckbehandlung von Wunden weiter zu verbessern und die Nachteile des Standes der Technik zu überwinden. Es sollen mit der vorliegenden Erfindung Vorrichtungen und Verfahren zur Unterdruckbehandlung von Wunden zur Verfügung gestellt werden, mit denen eine Behandlung möglichst wirksam und möglichst schonend durchgeführt werden kann.

Insbesondere soll die vorliegende Erfindung eine Unterdruckbehandlung von Wunden ermöglichen, die beim Patienten einen Wechsel der Wundauflagen in Intervallen von beispielsweise bis zu drei Tagen ermöglicht. Während eines Intervalls soll die Wundauflage vom Patienten subjektiv als angenehm empfunden werden. Druckreiz und Hautrötungen sollen vermieden werden. Beim Wechsel der Wundauflage nach einem Zeitraum von beispielsweise bis zu 3 Tagen sollen möglichst wenig unangenehme Gerüche auftreten. Die Keimdichte in der ausgewechselten Wundauflage soll niedrig sein.

Es soll eine Wundauflage bereit gestellt werden, die ein Verkleben mit der Abdeckfolie weitgehend vermeidet. Die Saugkraft soll nicht in Abhängigkeit vom Abstand zum Port abnehmen.

Die Aufgaben konnten unerwartet durch die Verwendung einer Wundauflage, die einen offenzelligen Schaumstoff enthält, der mit einer Salbengrundlage benetzt ist, gelöst werden. Überraschend wurde ebenfalls gefunden, dass eine Vorrichtung zur Unterdrucktherapie umfassend mindestens eine Wundauflage, die mit einer Salbengrundlage benetzt ist, zu einer vorteilhaften, d.h. sehr wirksamen und sehr schonenden Behandlung von Wunden besonders geeignet ist.

Es hat sich herausgestellt, dass die Aufgaben insbesondere vorteilhaft gelöst werden, wenn ein spezieller Schaumstoff und/oder eine spezielle Salbengrundlage verwendet werden und/oder wenn Art des Schaumstoffs und Art und Menge der Salbengrundlage gegenseitig aufeinander abgestimmt werden.

Gegenstand eines ersten Aspekts der Erfindung ist daher eine Vorrichtung zur Unterdrucktherapie von Wunden, umfassend,
(a) ein Abdeckmaterial zum luftdichten Verschließen eines Wundraums;
(b) gegebenenfalls ein Mittel zum Anschließen einer Unterdruckquelle; und
(c) eine Wundauflage, enthaltend
(c1) einen offenzelligen Schaumstoff, der mit
(c2) einer Salbengrundlage benetzt ist, die einen Tropfpunkt von 20 bis 80 °C aufweist.

Bevorzugt beträgt der Anteil an Salbengrundlage 10 bis 95 Gew.-% bezogen auf das Gesamtgewicht der Wundauflage.

Gegenstand eines zweiten Aspekts der Erfindung ist daher eine Vorrichtung zur Unterdrucktherapie von Wunden, umfassend,
(a) ein Abdeckmaterial zum luftdichten Verschließen eines Wundraums;
(b) gegebenenfalls ein Mittel zum Anschließen einer Unterdruckquelle; und
(c) eine Wundauflage, enthaltend
(c1) einen offenzelligen Schaumstoff, der mit
(c2) einer Salbengrundlage auf Basis eines Triglycerids benetzt ist.

Gegenstand eines dritten Aspekts der Erfindung ist daher eine Vorrichtung zur Unterdrucktherapie von Wunden, umfassend,
(a) ein Abdeckmaterial zum luftdichten Verschließen eines Wundraums;
(b) gegebenenfalls ein Mittel zum Anschließen einer Unterdruckquelle; und
(c) eine Wundauflage, enthaltend
(c1) einen speziellen offenzelligen Schaumstoff (siehe nachfolgende Erläuterungen zu Komponente (c1)), wobei der Schaumstoff (c1) insbesondere eine Luftdurchlässigkeit von 1000 bis 8000 1/(m²sec), gemessen gemäß DIN EN ISO 9237, aufweist, der mit (c2) einer Salbengrundlage benetzt ist. Bevorzugt beträgt der Anteil an Salbengrundlage 10 bis 95 Gew.-%, bezogen auf das Gesamtgewicht der Wundauflage.

Ebenfalls umfasst die Erfindung beliebige Kombinationen der genannten Aspekte.

Ferner ist Gegenstand der Erfindung die Verwendung eines offenzelligen Schaumstoffs, der mit einer Salbengrundlage benetzt ist, bevorzugt mit einer Salbengrundlage mit Tropfpunkt von 20 bis 80 °C, insbesondere mit einer Triglycerid-Salbengrundlage als Wundauflage zur oder in der Unterdrucktherapie von Wunden.

Ebenfalls ist Gegenstand der Erfindung ein Verfahren zur Herstellung einer Wundauflage, insbesondere zu einer in der Unterdrucktherapie verwendbaren Wundauflage, umfassend die Schritte
(I) Erwärmen einer Salbengrundlage über den Tropfpunkt,
(II) Einbringen eines offenzelligen Schaumstoffs in die erwärmte Salbengrundlage, so dass der Schaumstoff mit Salbengrundlage benetzt wird,
(III) gegebenenfalls temporäres Zusammendrücken des Schaumstoffs, bevorzugt auf mindestens 50 % und höchstens 90 % seines ursprünglichen Volumens, um eine vollständige Benetzung der inneren Oberfläche des Schaumstoffs mit der Salbengrundlage zu erreichen, und
(IV) gegebenenfalls Entfernen der überschüssigen Salbengrundlage, bevorzugt durch Ausdrücken des Schaumstoffs.

Die neue erfindungsgemäße Vorrichtung bzw. die erfindungsgemäße Verwendung der Wundauflage zeichnet sich durch mehrere unerwartete Vorteile aus.

Durch die Benetzung des Schaumstoffs mit der Salbengrundlage konnte die Anzahl der Partikel, welche unerwünscht in die Wunde gelangen, vorteilhaft reduziert werden.

Die Verwendung der erfindungsgemäßen Wundauflage verbesserte die atraumatischen Eigenschaften, so dass eine Unterdruckbehandlung ohne zusätzliche Wundkontaktschichten ermöglicht wurde.

Die Benetzung des Schaumstoffs mit der Salbengrundlage erniedrigte die subjektiv vom Patienten empfundene Härte des Schaumstoffs während der Unterdrucktherapie. Der Schaumstoff fühlte sich angenehmer an und die Patientencompliance (Befolgung der Therapieanweisung durch den Patienten) wurde erhöht.

Weiterhin hat sich gezeigt, dass trotz Einsatzes der Salbengrundlage die Saugkraft in Abhängigkeit vom Port nicht unerwünscht abnimmt. Überraschenderweise kann mit der erfindungsgemäßen Wundauflage sowohl eine ausreichende Durchleitung von Wundexsudat als auch ein die Wundheilung unterstützender Effekt erreicht werden, insbesondere bei Einsatz einer Salbengrundlage, welche Triglyceride und gegebenenfalls Diglyceride umfasst. Dies könnte auf der Aktivität der endogen in der Wunde vorhandenen Lipoproteinlipase beruhen. Die aus den Triglyceriden der Salbengrundlage freigesetzten Fettsäuren scheinen sich positiv auf die Wundheilung auszuwirken.

Ein die Wundheilung unterstützender Effekt kann bei der erfindungsgemäßen Wundauflage insbesondere dann erreicht werden, wenn durch die Wundauflage der Wunde eine ausreichende Menge an Salbe bereit gestellt wird. Eine zu große Menge an Salbengrundlage kann jedoch zu einer Verstopfung der Poren führen und somit die Durchleitung von Wundexsudat behindern. Bevorzugt beträgt der Anteil an Salbengrundlage 10 bis 95 Gew.-%, mehr bevorzugt 30 bis 92 Gew.-%, noch mehr bevorzugt 45 bis 90 Gew.-%, besonders bevorzugt 55 bis 88 Gew.-%, insbesondere 65 bis 85 Gew.-%, bezogen auf das Gesamtgewicht der Wundauflage.

Das erfindungsgemäße Herstellungsverfahren ist technisch einfacher und kann auch bei als Fertigprodukt bezogenen medizinischen Schaumstoffen eingesetzt werden. Es erfolgt eine gleichmäßige Benetzung, wodurch in allen Schaumstoffbereichen die Freisetzung von Partikeln reduziert wird wenn der Schaumstoff geschnitten wird. Im Gegensatz zu dem aus dem Stand der Technik bekannten Verfahren, bei welchem das Imprägnierungsmittel vor dem Aushärten bzw. der Synthese des Schaums zugegeben wird (siehe EP 0 335 669), ergeben sich keine unerwünschten Wechselwirkungen zwischen Salbengrundlage und Schaumstoff beim Aushärten oder der Synthese.

Ein weiterer Vorteil der erfindungsgemäßen Wundauflage besteht darin, dass ein Verkleben und/oder Verwachsen des Wundgrundes mit der Wundauflage selbst über einen Zeitraum von beispielsweise bis zu 3 Tagen weitgehend vermieden werden kann. Eine Traumatisierung der Wunde beim Verbandswechsel kann vermieden werden. Dies erhöht die Wirksamkeit der Wundbehandlung. Somit wird beim Patienten ein Wechsel der Wundauflagen in Intervallen von beispielsweise bis zu drei Tagen ermöglicht. Während des Intervalls von drei Tagen wurde die erfindungsgemäße Wundauflage vom Patienten als angenehm empfunden. Druckreiz und Hautrötungen wurden in der Regel vermieden. Beim Wechsel der Wundauflage nach einem Zeitraum von 3 Tagen traten wenig unangenehme Gerüche auf. Die Keimdichte in der ausgewechselten Wundauflage war unerwartet niedrig.

Die Bestandteile (a) bis (c) der erfindungsgemäßen Vorrichtung werden nachstehend beschrieben.

Die erfindungsgemäße Vorrichtung umfasst ein Abdeckmaterial (a) zum luftdichten Verschließen des Wundraums. Unter Wundraum wird die Wunde und gegebenenfalls die anliegende Wundumgebung verstanden. Unter "luftdichtem Verschließen" soll hier nicht verstanden werden, dass keinerlei Gasaustausch zwischen dem Wundraum und seiner Umgebung stattfindet. Vielmehr ist mit .,luftdichtem Verschließen" in diesem Zusammenhang gemeint, dass unter Berücksichtigung der eingesetzten Unterdruckpumpe der für die Unterdrucktherapie von Wunden nötige Unterdruck aufrecht erhalten werden kann. Es können deshalb auch Abdeckmaterialien eingesetzt werden, welche eine geringfügige Gaspermeabilität aufweisen, solange der für die Unterdrucktherapie notwendige Unterdruck aufrechterhalten werden kann.

In einer bevorzugten Ausführungsform der Erfindung umfasst das Abdeckmaterial zum luftdichten Verschließen der Wunde ein wasserunlösliches Polymer oder eine Metallfolie. Das Abdeckmaterial weist bevorzugt eine Dicke von 10 *µ*m bis 10.000 *µ*m*,* insbesondere von 25 *µm* bis 100 *µ*m*,* auf.

In einer bevorzugten Ausführungsform der Erfindung handelt es sich bei dem Abdeckmaterial (a) um ein wasserunlösliches Polymer. Bevorzugt weist das wasserunlösliche Polymer eine Löslichkeit von 10 mg/l oder weniger, mehr bevorzugt von 1 mg/ml oder weniger, insbesondere von 0,0001 bis 1 mg/ml auf (bestimmt gemäß Säulenelutionsmethode nach EU-Richtlinie RL67-548-EWG, Anhang V Kap. A6). Beispiele sind Polyurethan, Polyester, Polypropylen, Polyethylen, Polyamid oder Polyvinylchlorid, Polyorganosiloxan (Silikon) oder eine Mischung daraus. Die genannten Polymere liegen hierbei bevorzugt in nicht-zellulärer Form vor.

Es hat sich gezeigt, dass durch ein Abdeckmaterial mit einer speziellen Wasserdampfdurchlässigkeit die eingangs erläuterten Aufgaben besonders vorteilhaft gelöst werden können. In einer bevorzugten Ausführungsform weist daher das Abdeckungsmaterial einen Wasserdampfdurchlässigkeit von 100 bis 2500 g/m² x 24h, mehr bevorzugt von 500 bis 2000 g/m² x 24h, noch mehr bevorzugt von 800 bis 1600 g/m² x 24h, insbesondere von 1050 bis 1450 g/m² x 24h, bestimmt gemäß DIN EN 13726-2 bei 23 °C und 85 % relativer Luftfeuchte, auf. Insbesondere die Kombination einer Abdeckfolie (a) mit vorstehend genannter Wasserdampfdurchlässigkeit mit einem offenzelligen Schaumstoff (c1) mit nachstehend beschriebenen physikalischen Eigenschaften ist besonders vorteilhaft.

In einer bevorzugten Ausführungsform werden Abdeckmaterial und das Mittel zum Anschließen einer Unterdruckquelle bereits gebrauchsfertig miteinander verbunden zur Verfügung gestellt. Es ist ganz besonders bevorzugt, dass diese Ausführungsform eine Folie aus einem oder mehreren wasserunlöslichen Polymeren enthält, welche einen selbstklebenden Rand aufweist, da diese Anordnung das Anlegen des Verbandes wesentlich erleichtert.

Die erfindungsgemäße Vorrichtung zur Unterdrucktherapie umfasst gegebenenfalls ein Mittel (b) zum Anschließen einer Unterdruckquelle, d.h. ein Mittel zur Herstellung von Unterdruck im Wundraum. In einer bevorzugten Ausführungsform handelt es sich hierbei um ein Mittel (b) zur funktionellen Verbindung des Wundraums mit einer außerhalb des Abdeckmaterials befindlichen Unterdruckquelle, sodass ein Unterdruck im Wundraum herstellbar ist und Flüssigkeiten aus dem Wundraum absaugbar sind.

Der Ausdruck "Unterdruck im Wundraum" bezeichnet im Zusammenhang mit der Erfindung einen innerhalb des Wundverbandes gegenüber dem Umgebungsluftdruck (atmosphärischer Luftdruck) erniedrigten Luftdruck. Unter "innerhalb des Wundverbandes" wird der durch das Abdeckmaterial und die Wunde gebildete Zwischenraum verstanden.

Die Druckdifferenz zwischen dem Luftdruck innerhalb des Wundverbandes und dem Umgebungsluftdruck wird im Zusammenhang mit der Erfindung in mm Hg (Millimeter-Quecksilbersäule) angegeben, da dies im Bereich der Unterdrucktherapie üblich ist. 1 mm Hg entspricht einem Torr beziehungsweise 133,322 Pa (Pascal). Im Zusammenhang mit der Erfindung wird der Unterdruck, d. h. die Druckdifferenz zwischen dem Luftdruck innerhalb des Wundverbandes und dem Umgebungsluftdruck, als positiver Zahlenwert in mm Hg angegeben.

In einer Ausführungsform der Erfindung handelt es sich bei dem Unterdruck um einen Unterdruck von mindestens 25 mm Hg und höchstens 250 mm Hg, bevorzugt von mindestens 50 mm Hg und höchstens 150 mm Hg. Dieser Unterdruckbereich hat sich als für die Wundheilung vorteilhaft erwiesen. In einer bevorzugten Ausführungsform der Erfindung handelt es sich bei dem Unterdruck um einen Unterdruck von mindestens 80 mm Hg und höchstens 140 mm Hg, mehr bevorzugt von mindestens 120 mm Hg und höchstens 130 mm Hg.

Die erfindungsgemäße Vorrichtung zur Unterdrucktherapie von Wunden umfasst, wie vorstehend geschildert, bevorzugt ein Mittel (b) zum Anschließen einer Unterdruckquelle, d.h. ein Mittel zur funktionellen Verbindung des Wundraums mit einer außerhalb des Abdeckmaterials befindlichen Unterdruckquelle.

Die funktionelle Verbindung kann beispielsweise mit einer Verbindungsleitung oder mit einem Unterdruck-Anschlussstück hergestellt werden. Unterdruck-Anschlussstücke sind dem Fachmann auch unter der Bezeichnung "Port" bekannt.

Bei einer Ausführungsform handelt es sich bei dem Mittel (b) um eine Verbindungsleitung, bevorzugt einen Schlauch, insbesondere einen Silikon-Drainageschlauch. Die Verbindungsleitung kann durch das Abdeckmaterial hindurchgeführt werden. Alternativ kann die mindestens eine Verbindungsleitung unter dem Rand des Abdeckmaterials durchgeführt werden. In beiden Fällen ist die Durchtrittsstelle luftdicht abzudichten, sodass der gewünschte Unterdruck im Verband aufrecht erhalten werden kann. Als Abdichtmittel eignet sich beispielsweise eine Klebefolie, eine Klebemasse, oder ein Klebestreifen. Bei der Verbindungsleitung kann es sich beispielsweise um einen Schlauch, um ein Rohr oder um einen anderen Körper mit einem Hohlraum handeln.

In einer weiteren bevorzugten Ausführungsform handelt es sich bei dem Mittel (b) um ein Unterdruck-Anschlussstück (Port), welches auf einer der Innen- oder Außenseiten des Abdeckmaterials befestigt werden kann, wobei das Abdeckmaterial geeignete Öffnungen aufweist. Auch bei dieser Ausführungsform ist auf eine luftdichte Abdichtung entweder der Durchtrittsöffnung (Port innen) oder der Auflagefläche (Port außen) zu achten. Die Abdichtung kann beispielsweise mit einer Klebefolie, mit einer Klebemasse, oder mit einem Klebestreifen hergestellt werden. Es ist auch denkbar, dass der Port selbst über entsprechende Befestigungseinrichtungen, wie beispielsweise Klebeflächen, verfügt. Geeignete Unterdruck-Anschlussstücke sind kommerziell erhältlich. Dabei handelt es sich typischerweise um Unterdruck-Anschlussstücke, welche auf der Außenseite des Abdeckmaterials befestigt werden. Auch das Unterdruck-Anschlussstück verfügt zweckmäßigerweise über einen Unterdruckadapter, um mit den weiteren Komponenten des Unterdrucksystems verbindbar zu sein.

Neben den vorstehend beschriebenen Bestandteilen (a) und optional (b) umfasst die erfindungsgemäße Vorrichtung noch Bestandteil (c). Der Wundauflage (c), welcher in der erfindungsgemäßen Vorrichtung Verwendung findet, wird nachstehend genauer beschrieben. Alle Erläuterungen zur Wundauflage (c), inklusive der Bestandteile (c1) und (c2), betreffen nicht nur die erfindungsgemäße Vorrichtung, sondern auch das erfindungsgemäße Verfahren zur Herstellung der Wundauflage und die erfindungsgemäße Verwendung der Wundauflage in der Unterdrucktherapie.

Die Wundauflage (c) enthält einen offenzelligen Schaumstoff (c1) und eine Salbengrundlage (c2).

Als Schaumstoffe werden üblicherweise Werkstoffe mit über die gesamte Masse verteilten Zellen (offen, geschlossen oder beides) verstanden. Solche Werkstoffe weisen üblicherweise somit eine Rohdichte (gemäß DIN EN ISO 845) auf, die niedriger ist als die Dichte der Gerüstsubstanz.

Eine Zelle ist der bei der Herstellung von Schaumstoffen gebildete einzelne Hohlraum, der von Zellwänden und/oder Zellstegen teilweise oder vollständig umschlossen ist.

Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht. Im Rahmen dieser Anmeldung bedeutet der Begriff offenzellig, dass im Schaumstoff (c1) mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind. Die Offenzelligkeit des Polyurethanschaumstoffs wird üblicherweise nach ASTM D 2856-87, Verfahren B) bestimmt.

Unter Zellwand wird üblicherweise die die Zelle umschließende Wand verstanden. Die Zellwand kann auch als Zellmembran bezeichnet werden. Als Zellsteg wird üblicherweise der Bereich der Zellwand verstanden, der mehr als zwei Zellen voneinander trennt. Zellstege weisen bevorzugt mindestens das 1,5-fache der Dicke, mehr bevorzugt mindestens das 2-fache der Dicke, des übrigen Bereichs der Zellwand auf.

Bei dem offenzelligen Schaumstoff (c1) kann es sich um einen retikulierten Schaumstoff handeln. Unter einem retikulierten Schaumstoff wird ein Schaumstoff verstanden, der im Wesentlichen nur Zellstege aufweist. Bei einem retikulierten Schaumstoff sind die Zellwände somit im Wesentlichen entfernt. Die Retikulierung wird üblicherweise in einer Druckkammer, z.B. einer Stahlkammer, durchgeführt. Nach Einbringen des Schaumstoffs in die Stahlkammer wird die Luft abgesaugt (bevorzugt zu 50 bis 100 Gew.-%, mehr bevorzugt zu 70 bis 99 Gew.-%) und durch ein Brenngasgemisch, bevorzugt durch ein Gemisch enthaltend Wasserstoff und Sauerstoff, insbesondere im molaren Verhältnis von 2 : 1, ersetzt. Bei der Zündung des Gasgemisches zerreißen die Zellhäute durch die entstehende Hitze- und Druckwelle. Gegebenenfalls erfolgt auch ein zumindest teilweises Aufschmelzen der Zellstege, so dass diese verstärkt werden.

Der Schaumstoff (c1) weist bevorzugt eine Zellzahl (= Anzahl der Poren entlang einer Geraden pro laufendem cm) von 5 bis 25 cm-¹, mehr bevorzugt 7 bis 18 cm-¹, noch mehr bevorzugt 8 bis 15 cm⁻¹, auf. Die Zellzahl wird bevorzugt mikroskopisch bestimmt.

Grundsätzlich kann der offenzellige Schaumstoff aus beliebigem Material bestehen. Er sollte jedoch bestimmten physikalischen Anforderungen genügen. Es hat sich nämlich gezeigt, dass vorstehend genannte Aufgaben unerwartet vorteilhaft gelöst werden können, wenn der Schaumstoff (c1) eine spezielle Zugfestigkeit, eine spezielle Bruchdehnung und/oder einer spezielle Härte aufweist. In einer bevorzugten Ausführungsform weist der Schaumstoff (c1) eine Zugfestigkeit von 100 kPa bis 500 kPa, mehr bevorzugt 120 kPa bis 300 kPa, noch mehr bevorzugt 140 kPa bis 250 kPa, gemessen gemäß DIN 53571 (Probekörper A, Vorbehandlung im Normklima) auf. Ferner weist der Schaumstoff (c1) bevorzugt eine Bruchdehnung von 150 % bis 700 %, mehr bevorzugt von 200 % bis 650 %, noch mehr bevorzugt von 240 % bis 400 %, insbesondere 260 bis 320 %, gemessen gemäß DIN 53571 (Verfahren 1, Probekörper A), auf. Zudem weist der Schaumstoff (c1) bevorzugt eine Härte von 20 bis 70 Shore A, mehr bevorzugt von 30 bis 60 Shore A, noch mehr bevorzugt von 40 bis 50 Shore A, gemessen gemäß DIN 53505, wobei die Messung bei 23°C an einem plattenförmigen ebenen und glatten Probekörper einer Dicke von 6 mm erfolgte.

Es hat sich ferner gezeigt, dass vorstehend genannte Aufgaben unerwartet vorteilhaft gelöst werden können, wenn der Schaumstoff (c1) eine spezielle Luftdurchlässigkeit aufweist. In einer bevorzugten Ausführungsform weist der Schaumstoff (c1) eine Luftdurchlässigkeit von 1000 bis 8000 1/(m²sec), mehr bevorzugt von 1500 bis 6000 1/(m²sec), noch mehr bevorzugt von 2000 bis 5000 1/(m²sec), insbesondere von 2300 bis 4000 1/(m²sec), gemessen gemäß DIN EN ISO 9237 (20 mm Prüfdicke, 20 cm² Prüffläche , 200 Pa Differenzdruck) auf.

Es hat sich weiterhin gezeigt, dass vorstehend genannte Aufgaben unerwartet vorteilhaft gelöst werden können, wenn der Schaumstoff (c1) viskoelastisches Verhalten zeigt. Hierunter wird verstanden, dass das Verhalten des Schaumstoffs (c) auf Beanspruchung so aussieht, wie die Kombination aus einem elastischen Feststoff und einer viskosen Flüssigkeit. Das viskoelastische Verhalten kann durch einen Torsionsschwingungsversuch gemäß DIN 53445, Verfahren A, charakterisiert werden. Es ist bevorzugt, dass der Schaumstoff bei der Bestimmung gemäß DIN 53445, Verfahren A, bei 23 °C einen mechanischen Verlustfaktor von 0,1 bis 1,0, mehr bevorzugt von 0,15 bis 0,8, noch mehr bevorzugt von 0,2 bis 0,6, aufweist.

Es hat sich weiterhin gezeigt, dass vorstehend genannte Aufgaben unerwartet vorteilhaft gelöst werden können, wenn der Schaumstoff (c1) eine Rohdichte zwischen 15 und 55 kg/m³, mehr bevorzugt wischen 20 und 45 kg/m³, noch mehr bevorzugt zwischen 22 und 38 kg/m³, insbesondere zwischen 23 und 35 kg/m³ aufweist, gemessen gemäß DIN EN ISO 845 (Probekörper mit Abmessungen 100 mm x 100 mm x 50 mm, wobei die Messung bei Normklima, d.h. bei 23 °C und 50 % relativer Luftfeuchte, erfolgte und der Probenkörper vor der Messung 24 h auf Normklima konditioniert wurde).

In einer bevorzugten Ausführungsform ist der offenzellige Schaumstoff (c1) ausgewählt aus Polyurethanschaumstoffen (PUR), (bevorzugt quervernetzten) Polysiloxanschaumstoffen (SIL) und Polyvinylalkoholschaumstoffen (PVA). Polyurethanschaumstoffe sind besonders bevorzugt.

Bevorzugte Ausführungsformen der einsetzbaren Polyurethanschaumstoffe (c1-PUR) werden nachstehend erläutert. Der Polyurethanschaumstoff ist üblicherweise erhältlich durch Umsetzung einer härtbaren Mischung, umfassend die Komponenten
(i-PUR) Polyisocyanat,
(ii-PUR) gegenüber Isocyanat reaktive Verbindungen, insbesondere Polyol,
(iii-PUR), Katalysator,
(iv-PUR) Treibmittel, und
(v-PUR) gegebenenfalls Zusatzstoffe.

Als Isocyanate (1-PUR) können allgemein bekannte aliphatische, cycloaliphatische und/oder insbesondere aromatische Polyisocyanate eingesetzt werden. Zur Herstellung der Polyurethane eignen sich beispielsweise Diphenylmethandiisocyanat (MDI), hier insbesondere 4,4'- Diphenylmethandiisocyanat (4,4'-MDI), Mischungen aus monomeren Diphenylmethandiisocyanaten und höherkernigen Homologen des Diphenyhnethandüsocyanats (Polymer-MDI), Tetramethylendiisocyanat (TMDI), Hexamethylendiisocyanat (HDI), Toluylendiisocyanat (TDI) oder Mischungen daraus.

Bevorzugt wird MDI, insbesondere 4,4'-MDI und/oder HDI verwendet. Das besonders bevorzugt verwendete 4,4'-MDI kann geringe Mengen, bis etwa 10 Gew.-%, allophanat- oder uretonimin-modifizierte Polyisocyanate enthalten. Es können auch geringe Mengen Polyphenylenpolymethylenpolyisocyanat (PMDI) eingesetzt werden. Die Gesamtmenge dieser PMDI sollte 5 Gew.-% des eingesetzten Isocyanats nicht überschreiten.

Die Polyisocyanatkomponente (a) wird bevorzugt in Form von Polyisocyanatprepolymere eingesetzt. Diese Polyisocyanatprepolymere sind erhältlich, indem vorstehend beschriebene Polyisocyanate (i-PUR), beispielsweise bei Temperaturen von 30 bis 100 °C, bevorzugt bei etwa 80 °C mit einem Unterschuss an nachstehend beschriebenen Polyolen (ii-PUR) zum Prepolymer umgesetzt werden. Das Polyol-Polyisocyanat-Verhältnis wird hierbei so gewählt, dass der NCO-Gehalt des Prepolymeren 8 bis 28 Gew. -%, vorzugsweise 14 bis 26 Gew. -%, besonders bevorzugt 17 bis 23 Gew. -%, beträgt.

Als gegenüber Isocyanaten reaktive Verbindungen (ii-PUR) werden üblicherweise Polyole wie Polyetherole und/oder Polyesterole verwendet.

Möglich sind Polyetherpolyalkohole (in dieser Anmeldung als "Polyetherpolyole" bezeichnet) mit einer OH-Funktionalität von 1,9 bis 8,0, einer Hydroxylzahl von 50 bis 1000 mg KOH/g sowie gegebenenfalls 10 bis 100 % primären Hydroxylgruppen. Derartige Polyetherpolyole sind bekannt, kommerziell erhältlich, und basieren beispielsweise auf Starterverbindungen, die mit Alkylenoxiden, beispielsweise Propylenoxid und/oder Ethylenoxid, unter allgemein bekannten Bedingungen umgesetzt werden. Der Gehalt an primären Hydroxylgruppen kann erreicht werden, indem man die Polyole zum Abschluss mit Ethylenoxid umsetzt. Bevorzugt werden bei der Herstellung des offenzelligen Schaumstoffs (c) keine Polyetherpolyole verwendet.

Bevorzugt werden Polyesterpolyole in der Komponente (ii-PUR) verwendet. Die verwendeten Polyesterole (ii-PUR) werden im allgemeinen durch Kondensation von mehrfunktionellen Alkoholen, vorzugsweise Diolen, mit 2 bis 12 Kohlenstoffatomen, vorzugsweise 2 bis 6 Kohlenstoffatomen, mit mehrfunktionellen Carbonsäuren mit 2 bis 12 Kohlenstoffatomen, beispielsweise Bernsteinsäure, Glutarsäure, Adipinsäure, Phthalsäure, Isophthalsäure, und/oder Terephthalsäure und Gemischen hiervon, hergestellt. Beispiele für geeignete zwei- und mehrwertige Alkohole sind Ethandiol, Diethylenglykol, 1,4-Butandiol, 1,5-Pentandiol, und/oder 1,6-Hexandiol und Gemische hiervon.

Die Umsetzungsbedingungen von Carbonsäure und Alkohol werden üblicherweise so gewählt, dass die resultierenden Polyesterole keine freien Säuregruppen aufweisen. Ferner weisen die resultierenden Polyesterole im allgemeinen ein gewichtsmittleres Molekulargewicht (bestimmt mittels Gelpermeationschromatographie) von 500 bis 3000 g/mol, bevorzugt von mehr als 1000 g/mol bis 2500 g/mol, auf. Im allgemeinen weisen die eingesetzten Polyesterole eine mittlere theoretische Funktionalität von 2,0 bis 4, bevorzugt von mehr als 2 bis weniger als 3, auf. Des Weiteren weisen im allgemeinen die eingesetzten Polyesterole eine mittlere OH-Zahl von 20 bis 200, bevorzugt von 30 bis 90, auf.

In einer bevorzugten Ausführungsform weisen die verwendeten Polyesterole eine Viskosität von 150 mPa.s bis 600 mPa.s, bevorzugt von 200 mPa.s bis 550 mPa.s, mehr bevorzugt von 220 mPa.s bis 500 mPa.s, besonders bevorzugt von 250 mPa.s bis 450 mPa.s und insbesondere von 270 mPa·s bis 350 mPa.s, gemessen nach DIN 53 015 bei 75 °C, auf.

Die Verbindungen (ii-PUR) können in Mischung mit Kettenverlängerungs- und/oder Vernetzungsmitteln verwendet werden. Bei den Kettenverlängerungsmitteln handelt es sich überwiegend um 2-funktionelle Alkohole mit Molekulargewichten von 60 bis 499, beispielsweise Ethylenglykol, Propylenglykol, Butandiol-1,4, Pentandiol-1,5, Dipropylenglykol und/oder Tripropylenglykol. Bei den Vernetzungsmitteln handelt es sich um Verbindungen mit Molekulargewichten von 60 bis 499 und 3 oder mehr aktiven H-Atomen, vorzugsweise Aminen, und besonders bevorzugt Alkoholen, beispielsweise Glyzerin, Trimethylolpropan und/oder Pentaerythrit.

In einer bevorzugten Ausführungsform enthält (oder besteht aus) die Komponente (ii-PUR) 0-25 Gew.-%, bevorzugt 1 bis 20 Gew.-%, Kettenverlängerungs- und/oder Vernetzungsmittel und 75 bis 100 Gew.-%, bevorzugt 80 bis 99 Gew.-% Polyole(n), insbesondere Polyesterpolyole(n), bezogen auf das Gesamtgewicht der Komponente (ii-PUR)

Als Katalysatoren (iii-PUR) können übliche Verbindungen eingesetzt werden, welche die Reaktion der Komponente (i-PUR) mit der Komponente (ii-PUR) beschleunigen. In Frage kommen beispielsweise tertiäre Amine und/oder organische Metallverbindungen, insbesondere Zinnverbindungen. Beispielsweise können als Katalysatoren folgende Verbindungen eingesetzt werden: Triethylendiamin, Aminoalkyl- und/oder Aminophenylimidazole und/oder Zinn-(II)salze von organischen Carbonsäuren. Katalysatoren werden im allgemeinen in einer Menge von 0,1 bis 5 Gew.-% bezogen auf das Gewicht der Komponente (ii-PUR), eingesetzt.

Als Treibmittel (iv-PUR) können allgemein bekannte chemisch oder physikalisch wirkende Verbindungen eingesetzt werden. Als physikalisch wirkendes Treibmittel kann bevorzugt Wasser eingesetzt werden, welches durch Reaktion mit den Isocyanatgruppen Kohlendioxid bildet. Beispiele für physikalische Treibmittel sind (cyclo)aliphatische Kohlenwasserstoffe, vorzugsweise solche mit 4 bis 8, besonders bevorzugt 4 bis 6 und insbesondere 5 Kohlenstoffatomen, teilhalogenierte Kohlenwasserstoffe oder Ether, Ketone oder Acetate. Die Menge der eingesetzten Treibmittel richtet sich nach der angestrebten Dichte der Schaumstoffe. Die unterschiedlichen Treibmittel können einzeln oder in beliebigen Mischungen untereinander zum Einsatz kommen. Besonders bevorzugt wird nur Wasser als Treibmittel eingesetzt, im allgemeinen in einer Menge von 0,1 bis 5 Gew.-%, insbesondere von 2,5 bis 4 Gew.-%, bezogen auf das Gewicht der Komponente (ii-PUR). Physikalische Treibmittel werden bevorzugt in einer Menge von < 0,5 Gew.-%, bezogen auf das Gewicht der Komponente (ii-PUR), eingesetzt.

Die Umsetzung erfolgt gegebenenfalls in Anwesenheit von (v-PUR) Hilfs- und/oder Zusatzstoffen, wie z. B. Füllstoffen, Zellreglern, Zellöffnern, oberflächenaktiven Verbindungen und/oder Stabilisatoren gegen oxidativen, thermischen oder mikrobiellen Abbau oder Alterung.

Zur Herstellung von Polyurethanschaumstoffen werden im allgemeinen die Komponenten (i-PUR) und (ii-PUR) in solchen Mengen zur Umsetzung gebracht, dass das Äquivalenzverhältnis von NCO-Gruppen zur Summe der reaktiven Wasserstoffatome 1 :0,8 bis 1:1,25, vorzugsweise 1 :0,9 bis 1 :1,15, beträgt. Ein Verhältnis von 1:1 entspricht hierbei einem NCO-Index von 100. Die gewünschte Offenzelligkeit des Polyurethanschaums wird im allgemeinen durch eine dem Fachmann bekannte geeignete Wahl der Komponenten (i-PUR) bis (v-PUR) gewährleistet. Bevorzugt wird nach der Aushärtung der resultierende PUR-Schaumstoff retikuliert. Hierfür wird auf oben gegebene Erläuterungen verwiesen. Bevorzugte Ausführungsformen der einsetzbaren bevorzugt quervernetzten Polysiloxanschaumstoffe (cl-SIL) werden nachstehend erläutert.

Gemäß einer weiteren Ausführungsform handelt es sich bei dem offenzelligen Schaumstoff um einen Polysiloxan-Schaumstoff. Allgemein werden unter Polysiloxanen synthetische Polymere verstanden, bei denen Siliciumatome über Sauerstoffatome verknüpft sind.

In einer bevorzugten Ausführungsform ist der Schaumstoff (c1-SIL) erhältlich durch Umsetzung einer härtbaren Mischung, umfassend die Komponenten:
(i-SIL) ein Polyorganosiloxan enthaltend eine oder mehrere Gruppen mit einer C₂-C₆-Alkenylgruppe, bevorzugt enthaltend eine oder mehrere Vinyl-Gruppen,
(ii-SIL) ein Polyorganosiloxan enthaltend eine oder mehrere Si-H-Gruppen,
(iii-SIL) ein Treibmittel enthaltend eine oder mehrere OH-Gruppen, und
(iv-SIL) einen metallorganischen Katalysator.

In einer dritten Ausführungsform kann der offenzellige Schaumstoff ein Polyvinylalkohol-Schaumstoff (PVA-Schaumstoff) sein. Hierfür sind grundsätzlich die kommerziell erhältlichen PVA-Schaumstoffe geeignet, bevorzugt sollten sie jedoch die vorstehend genannten physikalischen Eigenschaften erfüllen.

Es hat sich weiterhin gezeigt, dass vorstehend genannte Aufgaben unerwartet vorteilhaft gelöst werden können, wenn der Schaumstoff (c1) Silber in Form von Silberionen oder in Form von atomarem Silber enthält. Bevorzugt wird nach der Herstellung des Schaumstoffs (c1) eine Silberbeschichtung aufgebracht. Alternativ kann das Silber bereits in die härtbare Zusammensetzung miteingebracht werden. Bevorzugt enthält der Schaumstoff (c1) 0,000001 bis 0,1 Gew.-%, mehr bevorzugt 0,0001 bis 0,01 Gew.-% Silber, bezogen auf das Gesamtgewicht des Schaumstoffs (c1).

In einer bevorzugten Ausführungsform der Erfindung weist der offenzellige Schaumstoff eine Dicke von 1 bis 50 mm, insbesondere von 15 mm bis 35 mm, auf.

In einer bevorzugten Ausführungsform wird der offenzellige Schaumstoff (c1) in trockenem Zustand mit der Salbengrundlage (c2) benetzt. Bevorzugt ist somit der Schaumstoff z.B. nicht mit einer Aktivierungslösung (wie z.B. Ringerlösung) getränkt.

Grundsätzlich sind als Salbengrundlage (c2) die im Fachgebiet bekannten und in Bauer, Frömming, Führer ,,Lehrbuch der Pharmazeutischen Technologie", 8. Auflage, Kapitel 12.1 bis 12.6 beschriebenen Salbengrundlagen geeignet. Demgemäß versteht man unter Salbengrundlagen streichfähige, halbfeste Zubereitungen, die grundsätzlich zur Anwendung auf der Haut oder auf den Schleimhäuten geeignet sind, jedoch (noch) keine pharmazeutischen Wirkstoffe enthalten. Die Herstellung von Salbengrundlagen ist dem Fachmann bekannt, es wird auf Ph. Eur. 6.0 "Halbfeste Zubereitungen zur kutanen Anwendung" verwiesen.

Bevorzugt werden Salbengrundlagen (c2) verwendet, die keine wässrige Phase enthalten. Bevorzugt werden hydrophobe, wasseraufnehmende und/oder hydrophile Salbengrundlagen verwendet. Hydrophobe Salbengrundlagen sind bevorzugt.

Hydrophobe Salbengrundlagen sind solche, die im Wesentlichen keine polaren Bestandteile enthalten und deshalb Wasser im Wesentlichen nicht aktiv binden. Es handelt sich daher um eine lipophile Grundlage, in die Wasser nur durch eine mechanische Dispergierung eingearbeitet werden kann. Beispiele für bevorzugte hydrophobe Salbengrundlagen sind Kohlenwasserstoffgrundlagen (z.B. Vaselin oder Vaselin-Paraffin-Mischungen), Diglyceride, Triglyceride, Wachse, Polyalkylsiloxane und Gemische daraus. Besonders bevorzugt werden Triglyceride oder Gemische aus Triglyceriden und Diglyceriden als Salbengrundlage (c2) verwendet.

Wasseraufnehmende Salbengrundlagen enthalten lipophile Stoffe und Tenside. Beispiele für wasseraufnehmende Salbengrundlagen sind W/O-Emulsionen (z.B. Wollwachs) oder O/W-Emulsionen.

Hydrophile Salbengrundlagen sind Zubereitungen, die mit Wasser mischbar sind. Bevorzugtes Beispiel ist eine Polyethylenglykol-Salbengrundlage (PEG mit gewichtsmittlerem Molekulargewicht von üblicherweise 300 bis 4000 g/mol, bevorzugt 1500 bis 3000 g/mol).

Neben den vorstehend beschriebenen bevorzugten Salbengrundlagen umfasst die Komponente (c2) auch noch Cremegrundlagen (hydrophile und hydrophobe Cremegrundlagen), Gele (hydrophobe Gele, hydrophile Gele) und Pasten (Suspensions-Salbengrundlagen).

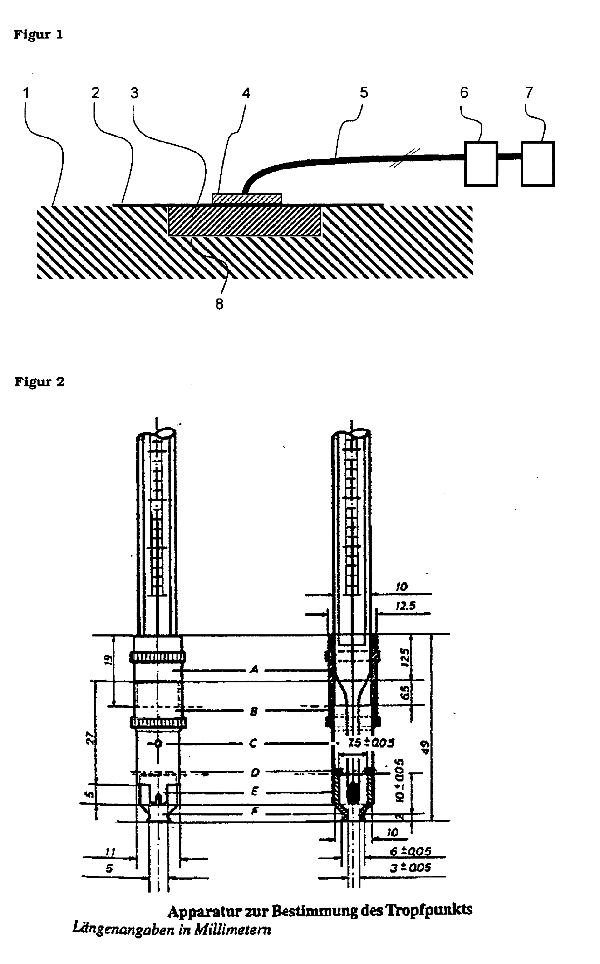

Die Salbengrundlage (c2) sollte von halbfester Konsistenz sein. Es hat sich zur Lösung der eingangs gestellten Aufgaben als vorteilhaft erwiesen, wenn die Salbengrundlage einen Tropfpunkt von 20 bis 80 °C, bevorzugt von 25 bis 55 °C, mehr bevorzugt von 30 bis 50 °C, noch mehr bevorzugt von 33 bis 48 °C und insbesondere von 35 bis 45 °C aufweist. Unter Tropfpunkt wird hierbei die Temperatur verstanden, bei welcher sich der erste Tropfen einer schmelzenden Substanz vom Metallnippel eines Tropfpunktthermometers löst. Zur experimentellen Bestimmung des Tropfpunkts wird auf die nachstehenden Erläuterungen zu Figur 2 verwiesen.

Neben dem Tropfpunkt hat es sich zur Lösung der eingangs beschriebenen Aufgaben als vorteilhaft erwiesen, wenn die Salbengrundlage (c2) einen oder mehrere der folgenden Parameter erfüllt:
Säurezahl von 0,001 bis 2,0 mg KOH/g, bestimmt gemäß Ph.Eur. 6.0, 2.5.1;
Iodzahl von 0,001 bis 3,0 g I2/100g, bestimmt gemäß Ph.Eur. 6.0, 2.5.4;
Peroxidzahl von 0,001 bis 1,0 mequi O/kg, bestimmt gemäß Ph.Eur. 6.0, 2.5.5 A; OH-Zahl von 1 bis 100, bevorzugt 5 bis 90 mg KOH/g, bestimmt gemäß Ph.Eur. 6.0, 2.5.3;
Verseifungszahl von 200 bis 350 mg KOH/g, bevorzugt von 240 bis 300 mg KOH/g, bestimmt gemäß Ph.Eur 6.0, 2.5.6;
Schwermetallgehalt von maximal 10 ppm, bestimmt gemäß Ph.Eur. 6.0, 2.4.8.D.

Sofern in den in dieser Anmeldung zitierten Normen und Ph.Eur.-Vorschriften nichts anderes angegeben ist, werden im Allgemeinen die Testmethoden bei Normklima, d.h. bei 23 °C und 50 % relativer Luftfeuchte, sowie bei einem Luftdruck von 1013 mbar durchgeführt.

In einer besonders bevorzugten Ausführungsform werden Triglyceride als Salbengrundlage (c2) verwendet. 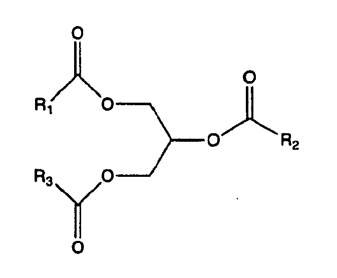 R₁, R₂ und R₃ können hierbei gleich oder bevorzugt unterschiedlich sein.

In einer bevorzugten Ausführungsform enthält das Triglycerid einen Glycerinrest und drei C₆-C₂₈-Säurereste, bevorzugt C₈-C₁₈-Säurereste. Die Säurereste können gesättigt oder ungesättigt sein, bevorzugt sind gesättigte Fettsäuren. Die Säurereste können gegebenenfalls substituiert sein, z.B. mit einer Hydroxygruppe.

Besonders bevorzugt enthalten die Säurereste der Triglyceride Caprylsäure, Caprinsäure, Laurinsäure und/oder Stearinsäure. Hierbei sind insbesondere Triglyceride bevorzugt, wobei die Säurefettfraktion 20 bis 40 Gew.-% Caprylsäure, 10 bis 30 Gew.-% Caprinsäure, 5 bis 20 Gew.-% Laurinsäure und 30 bis 50 Gew.-% Stearinsäure enthält, insbesondere daraus besteht.

In einer alternativen besonders bevorzugten Ausführungsform werden Diglyceride als Salbengrundlage (c2) verwendet. 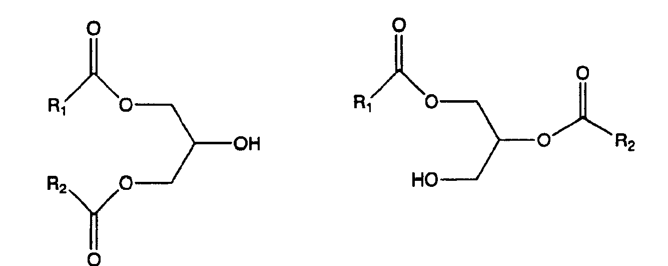 R₁ und R₂ können hierbei gleich oder bevorzugt unterschiedlich sein.

In einer bevorzugten Ausführungsform enthält das Diglycerid einen Glycerinrest und zwei C₄-C₂₈-Säurereste, bevorzugt C₆-Cₗ₈-Säurereste. Die Säurereste können gesättigt oder ungesättigt sein, bevorzugt sind gesättigte Fettsäuren. Die Säurereste können gegebenenfalls substituiert sein, z.B. mit einer Hydroxygruppe. Besonders bevorzugt enthalten die Säurereste der Isostearinsäure Stearinsäure, 12-Hydroxystearinsäure und/oder Adipinsäure.

In einer besonders bevorzugten Ausführungsform enthält die Salbengrundlage (c2) ein Gemisch aus Triglyceriden und Diglyceriden. Hierbei enthält die Salbengrundlage (c2) insbesondere
25 bis 90 Gew.-%, bevorzugt 45 bis 80 Gew.-% Triglyceride, und
10 bis 75 Gew.-%, bevorzugt 20 bis 55 Gew.-% Diglyceride.

Ebenfalls ist es bevorzugt, dass der Mischung aus Tri- und Diglyceriden noch Polyethylenglykol (PEG) zugesetzt wird. Insbesondere enthält die Mischung Tri-/DiGlyceride/PEG 1 bis 30 Gew.-%, bevorzugt 5 bis 20 Gew.-% Polyethylenglykol, bezogen auf das Gesamtgewicht der Mischung. Bevorzugt wird hierbei PEG mit einem gewichtsmittlerem Molekulargewicht von 500 bis 3000 g/mol, insbesondere von 1500 bis 2500 g/mol, verwendet.

Eine besonders bevorzugte Salbengrundlage enthält:
20 bis 90 Gew.-%, bevorzugt 55 bis 80 Gew.-% Triglyceride, insbesondere enthaltend Fettsäurereste, ausgewählt aus Caprylsäure, Caprinsäure, Laurinsäure und/oder Stearinsäure;
5 bis 75 Gew.-%, bevorzugt 15 bis 45 Gew.-% Diglyceride, insbesondere enthaltend Fettsäurereste, ausgewählt aus Isostearinsäure, Stearinsäure, 12-Hydroxystearinsäure und/oder Adipinsäure; und
0 bis 30 Gew.-%, bevorzugt 5 bis 20 Gew.-% Polyethylenglykol mit einem gewichtsmittleren Molekulargewicht von 500 bis 3000 g/mol.

Neben Di- und Triglyceriden kann die Salbengrundlage bevorzugt auch ferner Fettalkohole, alkoxylierte Fettalkohole, Fettsäuren und alkoxylierte Fettsäuren enthalten.

Die Salbengrundlage (c2) enthält bevorzugt keine Monoglyceride.

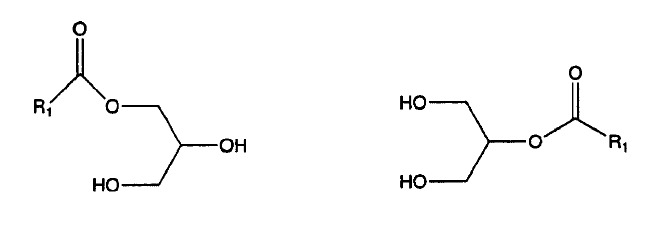

Insbesondere enthält die Salbengrundlage (c2) kein Glycerolmonooleat.

In einer bevorzugten Ausführungsform kann die Salbengrundlage (c2) auch Substanzen mit antimikrobieller Wirkung enthalten. Bei der Substanz mit antimikrobieller Wirkung kann es sich beispielsweise um Substanzen mit Amino oder Iminogruppen handeln. Weiterhin kann es sich bei der Substanz mit antimikrobieller Wirkung um antimikrobiell wirksame Metallkationen handeln, insbesondere um Silberkationen, beispielsweise um einen Komplex von 1-Vinyl-2-Pyrrolidone mit Silberkationen. Besonders geeignete Substanzen mit antimikrobieller Wirkung sind weiterhin Biguanid-Derivate wie Chlorhexidin oder Polybiguanide, wie Polyethylenbiguanid (PEB), Polytetramethylenbiguanid (PTMB) oder Polyethylenhexamethylenbiguanid (PEHMB). Ein besonders bevorzugtes Polybiguanid ist Polyhexamethylenbiguanid (PHMB bzw. Polyhexanid). Weitere geeignete Substanzen mit antimikrobieller Wirkung sind Polyguanidine wie zum Beispiel Polyhexamethylenguanidine (PHMG), N-Octyl - 1 - 10-(4-0ctyliminopyridin-1 - yl)decyl]pyridin-4-imin (Octenidin), quartäre Ammonium-verbindungen, wie zum Beispiel Benzalkoniumchlorid oder Cetylpyridiniumchlorid, Triazine wie zum Beispiel 1-(3-Chloroallyl)-3,5,7-triaza-1-azonia-adamantan-Chlorid oder die Ammoniumverbindung Taurolidin.

Substanzen mit antimikrobieller Wirkung sind üblicherweise in der Salbengrundlage (c2) in einer Menge von 0 bis 15 Gew.-%, bevorzugt von 0,1 bis 5 Gew.-%, bezogen auf das Gesamtgewicht der Salbengrundlage, enthalten.

Der offenzellige Schaumstoff (c1) wird mit der Salbengrundlage (c2) benetzt. Unter "benetzen" wird hierbei verstanden, dass die Salbengrundlage die äußeren und inneren Oberflächen (= die durch die offenen Poren gebildete innere Oberfläche) des Schaumstoffs bedeckt, bevorzugt vollständig bedeckt. In einer bevorzugten Ausführungsform werden mindestens 20 %, mehr bevorzugt mindestens 50 %, noch mehr bevorzugt mindestens 70 %, insbesondere mindestens 90 % der Gesamtoberfläche des Schaumstoffs (c1) mit Salbengrundlage bedeckt. Das Benetzen der Oberfläche des Schaumstoffs mit Salbengrundlage kann auch als Imprägnieren bezeichnet werden.

Es hat sich herausgestellt, dass durch eine spezielle Menge an Salbengrundlage die eingangs geschilderten Aufgaben besonders vorteilhaft gelöst werden können. In einer bevorzugten Ausführungsform beträgt der Anteil an Salbengrundlage (c2) 10 bis 95 Gew.-%, mehr bevorzugt 30 bis 92 Gew.-%, noch mehr bevorzugt 45 bis 90 Gew.-%, besonders bevorzugt 55 bis 88 Gew.-%, insbesondere 65 bis 85 Gew.-%, bezogen auf das Gesamtgewicht der Wundauflage (c). Das Gesamtgewicht der Wundauflage (c) ergibt sich aus der Addition der Gewichte der Bestandteile (c1) und (c2).

Grundsätzlich gilt, dass alle Erläuterungen zu bevorzugten Ausführungsformen einzelner Parameter des Polyurethanschaumstoffs (c1) und/oder der Salbengrundlage (c2) nicht isoliert zu betrachten sind, sondern auch in Kombination mit den Erläuterungen zu bevorzugten Ausführungsformen anderer Parameter bzw. in Kombination mit den Erläuterungen zu den Stoffzusammensetzungen von (c1) und (c2) gelten.

Die Wundauflage enthaltend (c1) und (c2) kann vorteilhaft durch das erfindungsgemäße Verfahren hergestellt werden. Dieses umfasst die Schritte:
(I) Erwärmen einer Salbengrundlage (c2) über den Tropfpunkt;
(II) Einbringen eines offenzelligen Schaumstoffs (c1) in die erwärmte Salbengrundlage, so dass der Schaumstoff mit Salbengrundlage benetzt wird,
(III) gegebenenfalls temporäres Zusammendrücken des Schaumstoffs, bevorzugt auf mindestens 50 % und höchstens 90 % seines ursprünglichen Volumens, um eine vollständige Benetzung der inneren Oberfläche des Schaumstoffs mit der Salbengrundlage zu erreichen, und
(IV) gegebenenfalls Entfernen der überschüssigen Salbengrundlage, bevorzugt durch Ausdrücken des Schaumstoffs.

In Schritt (I) wird die Salbengrundlage erwärmt, bevorzugt auf etwa 10 bis 30 °C über den Tropfpunkt.

In Schritt (II) wird der Schaumstoff eingebracht und bevorzugt untergetaucht. Die Verweilzeit in der Salbengrundlage ist abhängig von der Größe des Schaumstoffs und beträgt üblicherweise 10 bis 100 Sekunden.

Schritt (III) kann während oder nach, bevorzugt während dem Untertauchen erfolgen.

Unter temporäres Zusammendrücken des Schaumstoffs wird hier verstanden, dass der Schaumstoff manuell oder durch eine geeignete Maschine zusammengedrückt und danach wieder losgelassen wird. Dabei kann der Schaumstoff unmittelbar nach dem Zusammendrücken wieder losgelassen werden oder auch für kurze Zeit, beispielsweise für 1 bis 10 Sekunden, im zusammengedrückten Zustand gehalten und dann losgelassen werden. Nach dem auf das temporäre Zusammendrücken folgende Loslassen des Schaumstoffes kann der Schaumstoff dann wieder vollständig oder weitgehend sein ursprüngliches Volumen einnehmen. Während des Relaxierungsvorgangs wird üblicherweise Salbe aufgenommen, wobei eine vollständige Benetzung der inneren Oberfläche des Schaumstoffs mit der Salbengrundlage erreicht wird.

Anschließend wird der mit Salbengrundlage benetzte Schaumstoff entnommen. Überschüssige Salbengrundlage kann ausgedrückt werden (IV). Bevorzugt erfolgt das Ausdrücken bevor die Salbengrundlage unter den Tropfpunkt abgekühlt ist. Bevorzugt wird die Ausdrückkraft so gewählt, dass der Anteil an Salbengrundlage 10 bis 95 Gew.-% beträgt, mehr bevorzugt 30 bis 92 Gew.-%, noch mehr bevorzugt 45 bis 90 Gew.-%, besonders bevorzugt 55 bis 88 Gew.-%, insbesondere 65 bis 85 Gew.-%, bezogen auf das Gesamtgewicht der Wundauflage.

Alle vorstehend aufgeführten Erläuterungen zu bevorzugten Ausführungsformen der Komponenten (c), (c1) und (c2) finden auch für das erfindungsgemäße Verfahren Anwendung.

Weiterhin stellt die Erfindung ein gebrauchsfertiges Set zur Unterdruck-Wundbehandlung umfassend die erfindungsgemäße Vorrichtung, wobei der Schaumstoff (c1) als Wundauflage geeignet ist und gebrauchsfertig abgepackt vorliegt.

Gegenstand der Erfindung ist somit ein gebrauchsfertiges Set zur Unterdruck-Wundbehandlung, umfassend
(a) Abdeckmaterial zum luftdichten Verschließen eines Wundraums, d.h. der Wunde und der Wundumgebung,
(b) gegebenenfalls ein Mittel zum Anschließen einer Unterdruckquelle, bevorzugt ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind, und
(c) eine gebrauchsfertig abgepackte Wundauflage, enthaltend
(c1) einen offenzelligen Schaumstoff, der mit
(c2) einer Salbengrundlage benetzt ist, die bevorzugt einen Tropfpunkt von 20 bis 80 °C aufweist. Bevorzugt beträgt der Anteil an Salbengrundlage 10 bis 95 Gew.-%, bezogen auf das Gesamtgewicht der Wundauflage. Bevorzugt handelt es sich um eine Triglycerid-Salbengrundlage.

Die vom Set umfasste abgepackte Wundauflage (c) wird bevorzugt feuchtigkeitsdicht abgepackt. Vorzugsweise liegt die gebrauchsfertige Wundauflage in steriler Form vor, wobei die Sterilisation insbesondere durch Strahlen-Sterilisation erfolgen kann. Das Set kann weitere optionale Bestandteile wie beispielsweise Klebemittel zum Fixieren des Verbands, Abdichtmittel zur Herstellung einer luftundurchlässigen Abdichtung des Verbands, Drucksensoren, Verbindungselemente für Drucksensoren, zusätzliche Schläuche, Verbindungsstücke für Schläuche, Desinfektionsmittel, Hautpflegemittel, pharmazeutische Zubereitungen oder eine Gebrauchsanweisung enthalten. Bevorzugt enthält das erfindungsgemäße Set ferner Schere, Tupfer und/oder Pinzette, insbesondere in steriler Form.

Das Set kann sowohl die mindestens eine Wundkontaktschicht, als auch mindestens eine zusätzliche Druckverteilungsschicht umfassen. Vorzugsweise umfasst das Set weiterhin eine gebrauchsfertige Unterdruckeinheit.

Ein weiterer Gegenstand der Erfindung ist die Verwendung der vorstehend erläuterten Wundauflage (c) zur oder in der Unterdrucktherapie von Wunden. Gegenstand der Erfindung ist somit auch die Verwendung eines offenzelligen Schaumstoffs (c1), der mit einer Salbengrundlage (c2) benetzt ist, zur Unterdrucktherapie von Wunden, insbesondere als Wundauflage (c). Alle vorstehend aufgeführten Erläuterungen zu bevorzugten Ausführungsformen der Komponenten (c), (c1) und/oder (c2) finden sowohl einzeln als auch in Kombination auch für die erfindungsgemäße Verwendung Anwendung.

So betrifft beispielsweise die Erfindung die Verwendung eines Polyurethanschaumstoffs (c1), der erhältlich ist durch Umsetzung einer Mischung, umfassend die Komponenten
(i) Polyisocyanat,
(ii) Polyol, insbesondere Polyesterpolyol,
(iii) Treibmittel, und
(iv) Katalysator;
wobei bevorzugt der Schaumstoff erhältlich ist durch Umsetzung von einem Polyisocyanat (i), ausgewählt aus MDI, PMDI und/oder TDI, mit einem (ii) Polyesterpolyol, welches bevorzugt erhältlich ist durch Umsetzung einer Dicarbonsäure mit 4 bis 8 Kohlenstoffatomen mit einen Dialkohol mit 2 bis 6 Kohlenstoffatomen, wobei das (ii) Polyesterpolyol bevorzugt ein gewichtsmittleres Molekulargewicht von 500 bis 4000 g/mol aufweist;
wobei der offenzellige Polyurethanschaumstoff bevorzugt einen Aromatenanteil von 5 bis 50 %, mehr bevorzugt von 10 bis 45 %, insbesondere von 15 bis 40 %, aufweist; wobei der offenzellige Polyurethanschaumstoff bevorzugt eine Bruchdehnung von 150 % bis 700 %, mehr bevorzugt von 200 % bis 650 %, noch mehr bevorzugt von 240 % bis 400 %, insbesondere 260 % bis 320 %;
bevorzugt einen mechanischen Verlustfaktor von 0,1 bis 1,0, mehr bevorzugt von 0,15 bis 0,8, noch mehr bevorzugt von 0,2 bis 0,6;
wobei der offenzellige Polyurethanschaumstoff bevorzugt eine Härte von 20 bis 70 Shore A, mehr bevorzugt von 30 bis 60 Shore A, noch mehr bevorzugt von 40 bis 50 Shore A;
wobei der offenzellige Polyurethanschaumstoff bevorzugt eine Zellzahl (= Anzahl der Poren entlang einer Geraden pro laufendem cm) von 3 bis 40 cm⁻¹, bevorzugt 5 bis 25 cm⁻¹, mehr bevorzugt 7 bis 18 cm⁻¹, noch mehr bevorzugt 8 bis 15 cm⁻¹;
wobei der offenzellige Polyurethanschaumstoff bevorzugt eine Rohdichte zwischen 15 und 55 kg/m³, mehr bevorzugt zwischen 20 und 45 kg/m³, noch mehr bevorzugt zwischen 22 und 38 kg/m³, insbesondere zwischen 23 und 35 kg/m³; und/oder
wobei der offenzellige Polyurethanschaumstoff bevorzugt eine Luftdurchlässigkeit von 1000 bis 8000 1/(m²sec), mehr bevorzugt von 1500 bis 60001/(m²sec), noch mehr bevorzugt von 2000 bis 5000 1/(m²sec) und besonders bevorzugt von 2300 bis 40001/(m²sec) 2400 bis 3300 1/(m²sec) aufweist. In dieser erfindungsgemäßen Verwendung ist dieser Polyurethanschaumstoff (c1) mit einer Salbengrundlage (c2) benetzt, die einen Tropfpunkt von 20 bis 80 °C, bevorzugt von 25 bis 55 °C, mehr bevorzugt von 30 bis 50 °C, noch mehr bevorzugt von 33 bis 48 °C und insbesondere von 35 bis 45 °C aufweist;
die bevorzugt aus einer hydrophoben, Wasser aufnehmenden und/oder hydrophilen Salbengrundlage ausgewählt ist;
wobei die Salbengrundlage (c2) bevorzugt Triglyceride enthält und insbesondere ein Gemisch aus Triglyceriden und Diglyceriden, z.B. 25 bis 90 Gew.-%, bevorzugt 45 bis 80 Gew.-% Triglyceride, und 10 bis 75 Gew.-%, bevorzugt 20 bis 55 Gew.-% Diglyceride enthält.

Besondere Vorteile durch die erfindungsgemäße Vorrichtung, durch das erfindungsgemäße Set oder die erfindungsgemäße Verwendung, beziehungsweise Anwendung, ergeben sich, wenn es sich bei den Wunden um Brandwunden, um durch mechanische Traumatisierung entstandene Wunden, um eine durch Einwirkung von Chemikalien entstandene chronische Wunde, um eine durch eine Stoffwechselstörung verursachte chronische Wunde, um eine durch eine Durchblutungsstörungen verursachte chronische Wunde, oder um eine durch ein Druckgeschwür verursachte Wunde handelt.

In einer weiteren bevorzugten Ausführungsform wird die Wundauflage (c) (enthaltend (c1) und (c2)) zur Anwendung in der Unterdrucktherapie bei der Behandlung einer durch eine Hauttransplantation entstandenen Wunde bereitgestellt. Die Anwendung umfasst die Behandlung von durch Spalthaut-Transplantationen und von durch Vollhaut-Transplantationen entstandenen Wunden mittels Unterdrucktherapie. Vorteilhafte Wirkungen ergeben sich durch die Struktur des offenzelligen Schaumstoffs (c1), der mit der Salbengrundlage (c2) benetzt ist, sowie durch die gleichmäßige Druckverteilung. Bei der Anwendung der Wundauflage (c) bei der Behandlung einer durch eine Hauttransplantation entstandenen Wunde kann das Transplantat ("Skin-Graft") ausreichend fixiert und gleichzeitig können schädliche Scherkräfte vermieden werden.

Die vorstehend beschriebene Wundauflage (c) kann in der Unterdrucktherapie von Druckwunden bei Patienten mit einem Body-Mass-Index (BMI = Körpermasse durch Körperlänge im Quadrat) von weniger als 18,0, insbesondere mit einem Body-Mass-Index von 14 bis 17,5 vorteilhaft verwendet werden. Dies gilt insbesondere, wenn es sich um Patienten mit einem Alter von mehr als 60 Jahren handelt. Insbesondere bei diesen Patienten zeigt sich die vorteilhafte Wirkung der erfindungsgemäßen Vorrichtung oder des erfindungsgemäßen Sets.

Ein weiterer Gegenstand der Erfindung ist ein Verfahren zur Unterdrucktherapie von Wunden, umfassend die Schritte
a) Bereitstellen einer Vorrichtung nach einem der Ansprüche 1 bis 10;
b) Anlegen des Unterdruckverbandes an der Wunde;
c) Herstellung eines Unterdruckes von 25 mm Hg bis 250 mm Hg, bevorzugt 50 mm Hg bis 150 mm Hg im Wundraum für mindestens 30 Minuten und höchstens 7 Tage, bevorzugt für mindestens 1 Tag und höchstens 6 Tage.

### FIGUREN

- **Figur 1:**: schematischer Aufbau der erfindungsgemäßen Vorrichtung (Seitenansicht)
- **Figur 2:**: Apparatur zu Bestimmung des Tropfpunkts
- **Figur 3:**: Im Wundsimulator gemäß Beispiel 2 erhaltene Druckdaten

### Figurenlegende Figur 1:

- 1: Wundumgebung (d.h. in der Regel unversehrte Haut)
- 2: Luftundurchlässiges Abdeckmaterial (a)
- 3: Wundauflage (c) = offenzelliger Schaumstoff (c1), benetzt mit Salbengrundlage (c2)
- 4: Unterdruck- Anschlussstück (Port)
- 5: Unterdruck-Verbindungsleitung
- 6: Sammelgefäß
- 7: Unterdruckeinheit
- 8: Wundgrund

Die erfindungsgemäße Vorrichtung zur Unterdrucktherapie von Wunden wird anhand von **Figur 1** näher erläutert. Die Erfindung soll jedoch nicht auf die in den Zeichnungen oder in der Beschreibung der Zeichnungen dargestellten Ausgestaltungen reduziert verstanden werden. Vielmehr umfasst die erfindungsgemäße Vorrichtung auch Kombinationen der Einzelmerkmale der alternativen Formen.

In Figur 1 wird der schematische Aufbau der erfindungsgemäßen Vorrichtung in der Seitenansicht dargestellt. Die Vorrichtung umfasst ein luftundurchlässiges Abdeckmaterial (2), ein Mittel (4-5) zur funktionellen Verbindung des Wundraums mit einer außerhalb des Abdeckmaterials befindlichen Unterdruckquelle (7), sowie dem offenzelligen Schaumstoff (3). Das Abdeckmaterial (2) ist im Bereich der Wundumgebung (1), welche üblicherweise unversehrte Haut aufweist, befestigt. Die Größe des Abdeckmaterials sollte so bemessen sein, dass das Abdeckmaterial außerhalb des Wundbereichs im Bereich der Wundumgebung (1) befestigt werden kann. Das Abdeckmaterial (2) kann in verschiedenen Abmessungen und Formen vorliegen, beispielsweise kreisförmig, oval oder rechteckig. Es kann auch in einer an die Wunde angepassten, unregelmäßigen Form vorliegen. Das Abdeckmaterial (2) wird üblicherweise im Bereich der Wundumgebung (1) befestigt und luftdicht abgedichtet. Dies kann beispielsweise dadurch erfolgen, dass das Abdeckmaterial (2) einen Kleberand aufweist. Alternativ kann eine klebende Substanz entweder auf den Rand des Abdeckmaterials (2) und/oder auf die intakte Haut im Bereich der Wundumgebung aufgebracht werden. Dies hat den Vorteil, dass eine Anpassung des Abdeckmaterials an die Form und Größe der Wunde leichter möglich ist. Das Unterdruckanschlussstück (4) ist bei der hier gezeigten bevorzugten Ausführungsform auf der von der Wunde weg weisenden Außenseite des luftundurchlässigen Abdeckmaterials (2) angebracht. Um den Wundraum mit einer außerhalb des Abdeckmaterials befindlichen Unterdruckeinheit (7) funktionell zu verbinden, muss sich bei dieser Anordnung im Bereich des Unterdruckanschlussstücks (4) eine oder mehrere durch das Abdeckmaterial (2) hindurchgehende Öffnung(en) befinden.

Weiterhin ist eine luftdichte Abdichtung zu gewährleisten. Eine solche Abdichtung kann beispielsweise hergestellt werden, indem auf der von der Wunde weg weisenden Oberseite des Ports eine Folie (in Figur 1 nicht gezeigt) aufgebracht wird, die mit dem Abdeckmaterial (2) verklebt wird. Das Anlegen des Verbands kann erleichtert werden, wenn ein Port verwendet wird, bei dem ein geeignetes Befestigungs- und Abdichtmittel zum Befestigen des Ports auf dem Abdeckmaterial bereits vorhanden ist. Dies ist beispielsweise bei dem im Handel erhältlichen PPM-Drainageport^{®} der Firma Phametra-Pharma und Medica-Trading GmbH (Herne/Ruhrstadt, Deutschland) der Fall.

In einer bevorzugten Ausführungsform der Erfindung umfasst die Vorrichtung zur Unterdrucktherapie von Wunden keine Wundkontaktschicht zum Einbringen zwischen Wundauflage (3) und Wundoberfläche (8).
**Figur 2** zeigt die Apparatur zur Bestimmung des Tropfpunktes. Experimentell wird die Bestimmung wie folgt durchgeführt:

| | |
|---|---|
| Prüfgeräte: | - Tropfpunktthermometer nach Ubbelohde |
| | - 0 - 110 °C, geeicht |
| | - Becherglas 1000 ml |
| | - Reagenzglas, ca. 200 mm lang, Durchmesser: ≈ 40 mm, mit durchbohrten Stopfen |
| | - Magnetrührer mit Heizplatte |
| | - Filterpapierzuschnitte 10 x 10 mm |
| | |
| Reagenzien: | - demin. Wasser |
| | - Apparatur zur Bestimmung des Tropfpunktes gemäß Figur 2 |

Die Apparatur (siehe Figur 2) besteht aus 2 zusammengeschraubten Metallhülsen (A) und (B). Die Hülse (A) ist an einem Quecksilberthermometer befestigt. Ein Metallnippel (F) ist lose am unteren Teil der Hülse (B) mit 2 Klemmbacken (E) befestigt. Sperrstifte (D) von 2 mm Länge fixieren genau die Lage des Nippels. Sie dienen ebenfalls dem Zentrieren des Thermometers. Eine Öffnung (C) in der Wand der Hülse (B) dient als Druckausgleich.

Die Abtropffläche des Nippels muss plan und die Ränder der Austrittsöffnung müssen im rechten Winkel dazu sein. Der untere Teil des Quecksilberthermometers hat die Form und die Dimension wie in der Abbildung angegeben. Das Thermometer erlaubt Temperaturmessungen von 0 bis 110 °C, seine Skaleneinteilung beträgt 1 °C (je 1 mm). Das Quecksilbergefäß des Thermometers hat einen Durchmesser von 3,5 ± 0,2 mm und eine Höhe von 6,0 ± 0,3 mm.

Die ganze Apparatur wurde in die Mitte eines etwa 200 mm langen Reagenzglases von 40 mm äußerem Durchmesser mit Hilfe eines durchbohrten Stopfens, durch welchen das Thermometer gesteckt wurde, eingehängt. Der Stopfen hat an der Seite eine Einkerbung. Die Nippelöffnung muss 15 mm über dem Boden des Reagenzglases sein. Das Ganze wurde in ein mit Wasser gefülltes 1-Liter-Becherglas getaucht. Der Reagenzglasboden muss etwa 25 mm über dem Becherglasboden sein. Das Niveau des Wassers muss den oberen Teil der Hülse (A) erreichen. Ein Rührer sorgt für gleichmäßige Badtemperatur.

Das Prüfverfahren wurde wie folgt durchgeführt.

Der Nippel wurde vollständig mit der ungeschmolzenen zu prüfenden Substanz gefüllt, wenn nichts anderes vorgeschrieben ist. Mit einem Spatel wird der Substanzüberschuss an beiden Enden des Nippels abgestrichen. Die Hülsen (A) und (B) wurden zusammengeschraubt und der Nippel bis zu den Sperrstiften in die Hülse (B) eingeschoben. Die durch das Thermometer ausgestoßene Substanz an der Nippelöffnung wurde mit einem Spatel abgestrichen. Die Apparatur wurde, wie oben beschrieben, in das Wasserbad gehängt. Anschließend wurde das Wasserbad so erwärmt, dass von etwa 10 °C unterhalb des zu erwartenden Tropfpunktes an die Temperatur um etwa 1 °C je Minute stieg. Die Temperatur wurde abgelesen, als der erste Tropfen vom Nippel abfiel. Die Bestimmung wurde mindestens dreimal mit neuen Proben durchgeführt, dabei durften die einzelnen Werte höchstens 3 °C voneinander abweichen.

Auswertung: Als Tropfpunkt gilt der Mittelwert von drei Bestimmungen.

Die Erfindung soll durch nachfolgende Beispiele veranschaulicht werden.

### BEISPIELE

### Beispiel 1: Herstellung der erfindungsgemäßen Wundauflage (c)

Eine Salbengrundlage enthaltend eine Trigylcerid/Diglyceridmischung (Tropfpunkt ca. 40 °C) wurde auf 55 °C erwärmt. Ein offenzelliger Polyesterpolyol-Polyurethanschaumstoff (Rohdichte 28 kg/m³, Dicke 32 mm) wurde eingetaucht und auf ca. 70 % seines ursprünglichen Volumens zusammengepresst, so dass bei der auf das Zusammenpressen folgenden Ausdehnung des Schaums auf sein ursprüngliches Volumen auch die innere Oberfläche des Schaums vollständig mit Salbengrundlage benetzt wurde. Nach dem Herausnehmen wurde der Schaumstoff ausgedrückt, so dass der Anteil an Salbengrundlage nach dem Ausdrücken 79 Gew.-% betrug.

### Beispiel 2: Test der Wundauflage gemäß Beispiel 1 im Wundsimulator

Die Wundauflage gemäß Beispiel 1 wurde im Unterdruck-Wundsimulator (beschrieben in DE 10 2008 064 510 getestet.

Für eine "gut durchgeführte" Unterdrucktherapie-Simulation mussten die Ergebnisse des Tests lediglich sehr geringe Druckunterschiede zwischen dem Wundbereich (Sensor 1) und dem Portbereich (Sensor 2) sowie ein über einen langen Zeitraum gleichförmiges Verhalten aufweisen, bei gleichzeitiger linearer Ausführung bezüglich der entnommenen Exsudate. Der etwaige Druckunterschied ist auf den Wundverband zurückzuführen. Der Unterdruck wurde mittels eines ATMOS S041 Wunddrainagesaugers erzeugt. Das Exsudat wurde von einer B. Braun Perfusor F^{®} Spritzpumpe erzeugt, die einen konstanten Fluss erzeugen kann. Es wurde ein PPM Drainage Port-System (Firma Herne/Ruhrstadt, Deutschland) angewandt.

**Tabelle 1: Parameter**

| Wundverband | Temperatur (°C) | Druck (mm Hg) | Exsudat | | Wundgröße (cm²) |
|---|---|---|---|---|---|
| | | | Fluss (ml.h⁻¹) | Viskosität (mPa.s) (Albumin) | |
| erfindungsgemäße Wundauflage gemäß Beispiel 1 | 32 | 125 | 2 | 6,4 | 28,3 |

Die Wundauflage wurde auf dem Wundsimulator aufgebracht und mit Hydrofilm^{®} bedeckt, um ein luftdichtes System zu erzeugen. Über der künstlichen Wunde wurde ein kleines Loch in den Film geschnitten und ein PPM-Port wurde angebracht, damit das Exsudat aus dem Wundbereich herausfließen kann. Dieser Port wurde mit zum einen einem Behälter verbunden, um das Exsudat zu sammeln und zum anderen mit dem Druckmesser verbunden, um den Druck innerhalb des Port-Systems zu messen. Der Behälter wurde mit einer Vakuumpumpe verbunden und die Menge des extrahierten Exsudats gemessen, indem das Gewicht des Exsudats mittels einer Skala bestimmt wurde. Der andere Drucksensor, der innerhalb des Wundsimulators angebracht wurde, maß den Druck innerhalb der simulierten Wunde. Der Versuch wurde gemäß den Parameter in Tabelle 1 durchgeführt.

### Datenanalyse

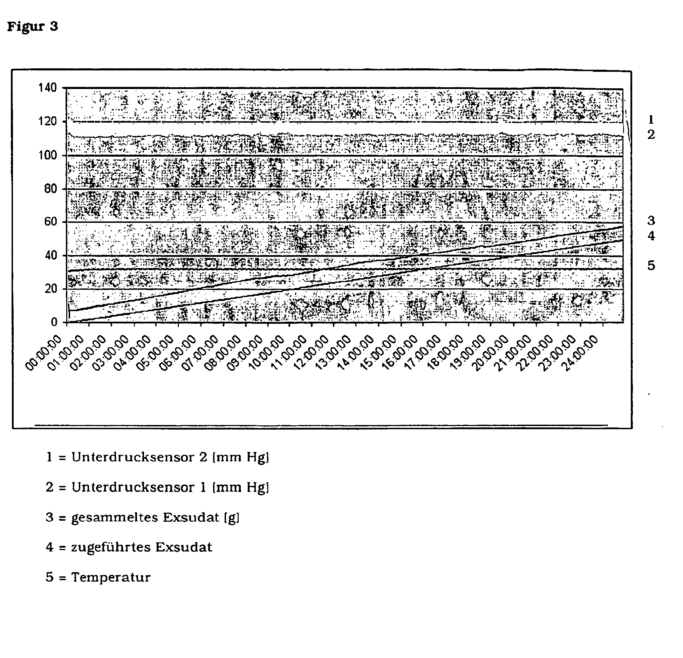

Der Versuch wurde über einen Zeitraum von 24 Stunden und 53 Minuten durchgeführt. Im nachfolgenden werden die Ergebnisse angegeben, die für die beiden oben genannten Drücke (Figur 3) erhalten wurden. Sensor 1 maß den Druck innerhalb der Wunde, während Sensor 2 den Druck innerhalb des Port-Systems bestimmte. Hierbei wurden Mittelwerte für die jeweiligen Drücke und deren Druckunterschiede erhalten (Tabelle 2).

**Tabelle 2**

| |
|---|
| Sensor 1 Mittelwert (mmHg): |
| 113,69 |
| |
| Sensor 2 Mittelwert (mmHg): |
| 115,90 |
| |
| Druckunterschied Mittelwert (mmHg): |
| **2,21** |

Die Ergebnisse zeigen, dass der Druckunterschied während des gesamten Versuchs gering und das Verhalten der Drücke normal blieb. Die errechneten und gesammelten Exsudate zeigten eine sehr ähnliche Kurve, was zudem den Erfolg des Versuchs bewies.

Daraus ergibt sich folgende Schlussfolgerung.

Die Menge des gesammelten Exsudats nahm gemäß den Erwartungen linear zu und der Druckunterschied zeigte sehr gute Ergebnisse. Dies bedeutet, dass die erfindungsgemäße Wundauflage gemäß Beispiel 1 in einem NPWT-Simulationsverfahren beständig und unerwartet vorteilhaft funktionierte.

## Patentansprüche

1. Vorrichtung zur Unterdrucktherapie von Wunden umfassend,
(a) ein Abdeckmaterial zum luftdichten Verschließen eines Wundraums;
(b) gegebenenfalls ein Mittel zum Anschließen einer Unterdruckquelle; und
(c) eine Wundauflage, enthaltend
(c1) einen offenzelligen Schaumstoff, der mit
(c2) einer Salbengrundlage benetzt ist, die einen Tropfpunkt von 20 bis 80 °C aufweist, wobei der Anteil an Salbengrundlage 10 bis 95 Gew.-% beträgt, bezogen auf das Gesamtgewicht der Wundauflage.

2. Vorrichtung nach Anspruch 1, wobei die Salbengrundlage (c2) ein Triglycerid enthält.

3. Vorrichtung nach Anspruch 2, wobei das in der Salbengrundlage (c2) verwendete Triglycerid einen Glycerinrest und drei C₆-C₂₈-Säurereste, bevorzugt C₈-C₁₈-Säurereste enthält.

4. Vorrichtung nach einem der vorangehenden Ansprüche, wobei die Salbengrundlage (c2) ein Diglycerid enthält.

5. Vorrichtung nach Anspruch 5, wobei das in der Salbengrundlage (c2) verwendete Diglycerid einen Glycerinrest und zwei C₄-C₂₈-Säurereste, bevorzugt C₆-C₁₈-Säurereste enthält.

6. Vorrichtung nach einem der vorhergehenden Ansprüche, wobei es sich bei dem Schaumstoff (c1) um einen Polyurethanschaumstoff handelt, der erhältlich ist durch Umsetzung einer Mischung, umfassend die Komponenten
(i) Polyisocyanat,
(ii) Polyol, insbesondere Polyesterpolyol,
(iii) Treibmittel, und
(iv) Katalysator.

7. Vorrichtung nach einem der vorangehenden Ansprüche, wobei der Schaumstoff (c1) eine Zugfestigkeit von 100 kPa bis 500 kPa, gemessen gemäß DIN 53571 und/oder eine Bruchdehnung von 200 % bis 700 %, jeweils gemessen gemäß DIN 53571, aufweist.

8. Vorrichtung nach einem der vorangehenden Ansprüche, wobei der Schaumstoff (c1) eine Luftdurchlässigkeit von 1000 bis 8000 l/(m²sec), gemessen gemäß DIN EN ISO 9237, aufweist.

9. Vorrichtung nach einem der vorangehenden Ansprüche, wobei Schaumstoff (c1) eine Rohdichte zwischen 15 und 40 kg/m³ aufweist, gemessen gemäß DIN EN ISO 845.

10. Vorrichtung nach einem der vorangehenden Ansprüche, wobei das Abdeckmaterial (a) eine Wasserdampfdurchlässigkeit von 100 bis 2500 g/m² x 24 h aufweist, gemessen gemäß DIN EN 13726-2.

11. Verfahren zur Herstellung einer Wundauflage, umfassend die Schritte
(I) Erwärmen einer Salbengrundlage über den Tropfpunkt;
(II) Einbringen eines offenzelligen Schaumstoffs in die erwärmte Salbengrundlage, so dass der Schaumstoff mit Salbengrundlage benetzt wird;
(III) gegebenenfalls temporäres Zusammendrücken des Schaumstoffs, bevorzugt auf mindestens 50 % und höchstens 90 % seines ursprünglichen Volumens, um eine vollständige Benetzung der inneren Oberfläche des Schaumstoffs mit der Salbengrundlage zu erreichen; und
(IV) gegebenenfalls Entfernen der überschüssigen Salbengrundlage, bevorzugt durch Ausdrücken des Schaumstoffs.

12. Verfahren gemäß Anspruch 11, wobei die Ausdrückkraft so gewählt wird, dass der Anteil an Salbengrundlage 10 bis 95 Gew.-% beträgt, bezogen auf das Gesamtgewicht der Wundauflage.

13. Verwendung eines offenzelligen Schaumstoffs, der mit einer Triglycerid-Salbengrundlage benetzt ist, als Wundauflage zur Unterdrucktherapie von Wunden.

14. Verwendung gemäß Anspruch 13, wobei es sich um Brandwunden, um durch mechanische Traumatisierung entstandene Wunden, um durch Einwirkung von Chemikalien entstandene Wunden, um durch Stoffwechselstörungen verursachte Wunden, um durch Durchblutungsstörungen verursachte Wunden oder um durch Druckgeschwüre verursachte Wunden handelt.

## Claims

1. Device for negative pressure wound therapy comprising
(a) a cover material for air-tight sealing of the wound space;
(b) optionally, a means for the connection of a negative pressure source; and
(c) a wound dressing containing
(c1) an open-cell foam which is
(c2) wetted with an ointment base which has a dropping point of 20 to 80°C, wherein the proportion of ointment base is 10 to 95 weight percent relative to the total weight of the wound dressing.

2. Device in accordance with claim 1, wherein the ointment base (c2) contains a triglyceride.

3. Device in accordance with claim 2, wherein the triglyceride used in the ointment base (c2) contains a glycerin residue and three C₆-C₂₈ acid residues, preferably C₈-C₁₈ acid residues.

4. Device in accordance with one of the previous claims, wherein the ointment base (c2) contains a diglyceride.

5. Device in accordance with claim 4, wherein the diglyceride used in the ointment base (c2) contains a glycerin residue and two C₄-C₂₈ acid residues, preferably C₆-C₁₈ acid residues.

6. Device in accordance with one of the previous claims, wherein the foam (c1) is a polyurethane foam which can be obtained by reaction of a mixture comprising the components
(i) polyisocyanate,
(ii) polyol, in particular polyester polyol,
(iii) blowing agent, and
(iv) catalyst.

7. Device in accordance with one of the previous claims, wherein the foam (c1) has a tensile strength of 100 kPa to 500 kPa, measured in accordance with DIN 53571 and/or a ductile yield of 200% to 700%, each measured in accordance with DIN 53571.

8. Device in accordance with one of the previous claims, wherein the foam (c1) has an air permeability of 1,000 to 8,000 l/(m²sec), measured in accordance with DIN EN ISO 9237.

9. Device in accordance with one of the previous claims, wherein the foam (c1) has a raw density between 15 and 40 kg/m³, measured in accordance with DIN EN ISO 845.

10. Device in accordance with one of the previous claims, wherein the cover material (a) has a water vapor permeability of 100 to 2,500 g/m² x 24 h, measured in accordance with DIN EN 13726-2.

11. Method for the manufacture of a wound dressing comprising the steps of
(I) heating an ointment base above the dropping point,
(II) introducing an open-cell foam into the heated ointment base so that the foam is wetted with the ointment base,
(III) optionally, temporarily compressing the foam, preferably to at least 50% and up to a maximum of 90% of its original volume in order to ensure that the internal surface of the foam is fully wetted with the ointment base, and
(IV) optionally, removing the surplus ointment base, preferably by squeezing the foam.

12. Method in accordance with claim 11, wherein the compression force is selected in such a way that the proportion of ointment base is 10 to 95 weight per cent relative to the total weight of the wound dressing.

13. Use of an open-cell foam wetted with a triglyceride ointment base as a wound dressing for the negative pressure wound therapy.

14. Use in accordance with claim 13, wherein the wounds are bum wounds, wounds caused by mechanical trauma, wounds caused by exposition to chemicals, wounds caused by a metabolic disorder, wounds caused by a circulatory disorder or wounds caused by pressure ulcers.

## Revendications

1. Dispositif permettant de soigner des plaies par application d'une pression négative, comprenant
(a) un matériau de couverture destiné à fermer un espace de plaie de manière à le rendre étanche à l'air ;
(b) le cas échéant un moyen permettant le raccordement à une source de pression négative ; et
(c) une couche de contact avec la plaie, contenant
(c1) une mousse à pores ouverts laquelle est mouillée avec
(c2) une base de pommade présentant un point de goutte compris entre 20 et 80 °C, la proportion de la base de pommade étant comprise entre 10 et 95 % en poids, par rapport au poids total de la couche de contact avec la plaie.

2. Dispositif selon la revendication 1, la base de pommade (c2) contenant un triglycéride.

3. Dispositif selon la revendication 2, le triglycéride utilisé dans la base de pommade (c2) contenant un résidu de glycérol et trois résidus d'acides en C₆-C₂₈, s'agissant préférentiellement de résidus d'acides en C₈-C₁₈.

4. Dispositif selon l'une des revendications précédentes, la base de pommade (c2) contenant un diglycéride.

5. Dispositif selon la revendication 4, le diglycéride utilisé dans la base de pommade (c2) contenant un résidu de glycérol et deux résidus d'acides en C₄-C₂₈, s'agissant préférentiellement de résidus d'acides en C₆-C₁₈.

6. Dispositif selon l'une des revendications précédentes, la mousse (c1) étant une mousse de polyuréthane pouvant être obtenue en faisant réagir un mélange contenant les constituants suivants
(i) un polyisocyanate,
(ii) un polyol, notamment un polyesterpolyol,
(iii) un agent gonflant, et
(iv) un catalyseur.

7. Dispositif selon l'une des revendications précédentes, la mousse (c1) présentant une résistance à la traction comprise entre 100 kPa et 500 kPa, mesurée selon la norme DIN 53571, et/ou un allongement à la rupture compris entre 200 % et 700 %, les deux étant mesurés selon la norme DIN 53571.

8. Dispositif selon l'une des revendications précédentes, ladite mousse (c1) présentant une perméabilité à l'air, mesurée selon la norme DIN EN ISO 9237, comprise entre 1000 et 8000 l/(m²sec).

9. Dispositif selon l'une des revendications précédentes, la mousse (c1) présentant une densité volumétrique, mesurée selon la norme DIN EN ISO 845, comprise entre 15 et 40 kg/m³.

10. Dispositif selon l'une des revendications précédentes, le matériau de couverture (a) présentant une perméabilité à la vapeur d'eau, mesurée selon la norme DIN EN 13726-2, comprise entre 100 et 2500 g/m² x 24 h.

11. Procédé de fabrication d'une couche de contact avec la plaie, comprenant les étapes suivantes
(I) réchauffement d'une base de pommade jusqu'à dépasser son point de goutte ;
(II) incorporation d'une mousse à pores ouverts dans la base de pommade réchauffé, faisant en sorte que la base de pommade vienne mouiller ladite mousse ;
(III) le cas échéant, compression temporaire de la mousse, de préférence jusqu'à ce que son volume soit compris entre 50 % et 90 % de son volume initial, pour faire en sorte que la base de pommade vienne mouiller la totalité de la surface intérieure de la mousse ; et
(IV) le cas échéant, enlèvement de l'excès de base de pommade, de préférence en exerçant une pression sur la mousse.

12. Procédé selon la revendication 11, la force de pression étant choisie telle que la proportion de la base de pommade est comprise entre 10 et 95 % en poids, par rapport au poids total de la couche de contact avec la plaie.

13. Utilisation d'une mousse à pores ouverts, laquelle est mouillée avec une base de pommade aux triglycérides, en tant que couche de contact avec la plaie destinée à soigner des plaies par application d'une pression négative.

14. Utilisation selon la revendication 13, s'agissant de brûlures, de plaies provoquées par un trauma mécanique, de plaies provoquées par l'action de produits chimiques, de plaies provoquées par des perturbations du métabolisme, de plaies provoquées par des troubles de la circulation sanguine ou de plaies provoquées par des ulcères résultant d'une pression.
